**Europäisches Patentamt**

**European Patent Office**

**Office européen des brevets**

(19)

(11) Veröffentlichungsnummer: **0 154 865**
**B1**

(12) **EUROPÄISCHE PATENTSCHRIFT**

(45) Veröffentlichungstag der Patentschrift:
**19.11.87**

(51) Int. Cl.⁴: **A 61 B 17/36,** G 05 D 3/14,
G 02 B 7/24, G 02 B 26/08

(21) Anmeldenummer: **85101962.0**

(22) Anmeldetag: **22.02.85**

(54) **Einrichtung zur Lagekorrektur eines über eine Gelenkoptik geführten Laserstrahls.**

(30) Priorität: **24.02.84 DE 3406676**

(43) Veröffentlichungstag der Anmeldung:
**18.09.85 Patentblatt 85/38**

(45) Bekanntmachung des Hinweises auf die Patenterteilung:
**19.11.87 Patentblatt 87/47**

(84) Benannte Vertragsstaaten:
**CH DE FR GB LI SE**

(56) Entgegenhaltungen:
**EP - A - 0 048 364**
**DE - A - 2 645 393**
**DE - A - 3 202 432**
**DE - B - 2 757 585**
**US - A - 4 129 775**
**US - A - 4 473 074**

**PATENT ABSTRACTS OF JAPAN, Band 7, Nr. 6, 11.**
**Januar 1983, Seite (P-167) (1151)**
**PATENT ABSTRACTS OF JAPAN, Band 5, Nr. 131, 21.**
**August 1981, Seite (M-84) (803)**

(73) Patentinhaber: **Firma Carl Zeiss, D-7920 Heidenheim**
**(Brenz) (DE)**
Patentinhaber: **CARL-ZEISS-STIFTUNG trading as CARL**
**ZEISS, D-7920 Heidenheim (Brenz) (DE)**

(72) Erfinder: **Müller, Gerhard, Prof. Dr.,**
**Philipp-Funk-Strasse 112, D-7080 Aalen (DE)**
Erfinder: **Hohberg, Gerhard, Dr., Kopernikusstrasse 34,**
**D-7082 Oberkochen (DE)**
Erfinder: **Greve, Peter, Dr., Teussenbergweg 13,**
**D-7081 Essingen (DE)**

## Beschreibung

Zur Führung von Laserstrahlen verwendet man entweder Lichtleiter in Form von flexiblen Glasfasern oder Übertragungssysteme, die ablenkende Optik, in der Regel Spiegel, enthalten. Insbesondere für medizinische Anwendungsfälle wie z.B. die Laserchirurgie werden oft gelenkige Übertragungssysteme des letztgenannten Typs eingesetzt, da es bisher keine Faserkabel gibt, die genügend bruchsicher sind und gleichzeitig im Wellenlängenbereich der dort meist verwendeten $CO_2$-Laser ausreichend verlustarm übertragen.

Die zur Führung des Lasers benutzten Gelenkarme bestehen aus einer Reihe von Umlenkspiegeln, die an Drehgelenken derart befestigt sind, dass jeder Spiegel mitsamt dem darauffolgenden Teil des Gelenkarms um die Achse des jeweils einfallenden Laserstrahls drehbar ist. Zusätzlich sind zwischen den Gelenken oft Teleskopführungen eingesetzt, die es erlauben die Länge des Arms zu verändern. Bekannte Gelenkarme besitzen bis zu sieben Drehgelenke.

Die einwandfreie Funktion eines solchen Gelenkarms hängt abgesehen von einer genügend genauen Justage der Spiegel von der Qualität der Drehlager bzw. Teleskopführungen sowie der Steifigkeit ihrer Verbindungen ab. Die dabei auftretenden Probleme wachsen mit den Abmessungen des vom Gelenkarm überstreichbaren Arbeitsbereiches. Die Erhöhung der Steifigkeit des Arms erfordert stärkeres Material und demzufolge grössere zu bewegende Massen, was die Drehlager stärker belastet und vor allem im dynamischen Betrieb die Handhabung beeinträgt.

Treten jedoch Führungsfehler auf, dann wandert de Strahl aus. In erster Linie verlagert sich dabei der Fokus des Lasers, was in gewissen Grenzen insbesondere, wenn die Fokuslage über einen eingespiegelten Pilotstrahl visuell beurteilt wird, nicht sonderlich stört. Bei langen Gelenkarmen kann jedoch der Fall eintreten, dass der Laserstrahl innerhalb der Gelenkoptik aus den freien Öffnungen der Umlenkspiegel oder der Fokusoptik auswandert und auf deren Fassungen trifft. Bei Hochleistungslasern ist damit eine Zerstörung der Optik verbunden.

Zwar ist es bei Lasersystemen zum Auslesen von Informationsspeicherplatten bekannt, Regeleinrichtungen vorzusehen, die den Fokus des Laserstrahls radial einer vorgegebenen Spur nachführen. Diese Regeleinrichtungen werten jedoch die Ablage des Fokus von der sichtbar vorgezeichneten Datenspur aus. Ausserdem besteht eine feste räumliche Zuordnung zwischen der Bewegungsrichtung des Stellglieds und der des Laserfokus. Ist der Laserstrahl jedoch über mehrere Drehgelenke geführt, so muss bei der Bestimmung der Zuordnung zwischen den Positionskoordinaten des Fokus und den Stellkoordinaten des nachführenden Elements der Drehwinkel jedes Gelenks berücksichtigt werden. Dies ist jedoch nicht ohne weiteres möglich, denn würde man jedem Gelenk einen eigenen Winkelgeber zuordnen, ergäbe sich insbesondere bei Gelenkarmen mit mehreren Drehachsen ein beträchtlicher zusätzlicher Aufwand.

Die Aufgabe der vorliegenden Erfindung ist es, bei möglichst geringem Aufwand eine Gelenkoptik für einen Leistungslaser zu schaffen, die leicht baut und ohne übermässige Anforderungen an die Qualität der verwender Lager eine ausreichend stabile Strahlführung ermöglicht.

Diese Aufgabe wird gemäss dem Kennzeichen des Hauptanspruches dadurch gelöst, dass der Laserstrahl vor Eintritt in die Gelenkoptik über ein die Strahlrichtung mit kleiner Amplitude modulierendes, optisches Element geführt, ein Teil der Laserstrahlung auf einen am Ausgang der Gelenkoptik angeordneten, positionsempfindlichen Detektor gerichtet und eine Schaltung vorgesehen ist, der das Signal des Detektors zur Bildung eines Regelsignals für einen am Eingang der Gelenkoptik angeordneten, steuerbaren Strahlablenker zugeführt ist.

Gemäss der Erfindung werden also am Ausgang der Gelenkoptik festgestellte Abweichungen des Laserstrahls von der Achse der Gelenkoptik über einen am Eingang angeordneten Strahlablenker kompensiert. Demzufolge können was die Steifigkeit und die Qualität der Lager des Arms betrifft Abstriche gemacht werden und durch Verminderung der Anforderung an die Führungsgenauigkeit Kosten eingespart werden, die den zusätzlichen Aufwand für die Elemente zur Regelung der Strahlposition weit übertreffen.

Da die Modulation der Strahlrichtung am Eingang der Gelenkoptik auf den am Ausgang angeordneten Empfänger Wechselsignale erzeugt, deren Amplitudenverhältnisse bzw. Phasenlage eine eindeutige Richtungszuordnung ermöglichen, ist immer eine korrekte Nachregelung der Strahlposition trotz der im Gelenkarm auftretenden Drehbewegungen möglich und zwar unabhängig von der Anzahl der verwendeten Drehgelenke.

Es ist möglich das die Strahlrichtung modulierende Element, das vorzugsweise aus einem piezoelektrisch auslenkbaren Spiegel besteht, gleichzeitig als Stellglied für die Regelung der Strahlposition zu verwenden, indem dem Wechselsignal auf der Modulationsfrequenz das Regelsignal überlagert wird.

Wird jedoch koxial zum Strahl des eigentlichen Arbeitslasers ein Hilfs- oder Pilotlaser eingespiegelt, der ohnehin zur visuellen Erkennung des Fokus verwendet wird, dann ist es zweckmässig das modulierende optische Element im Strahl des Pilotlasers vor dessen Einkopplung in den Strahl des Arbeitslasers anzuordnen und ein separates Stellglied im gemeinsamen Strahlengang beider Laser vorzusehen. Als positionsempfindliche Detektoren können vorteilhaft bekannte photoelektrische Quadrantenempfänger verwendet werden.

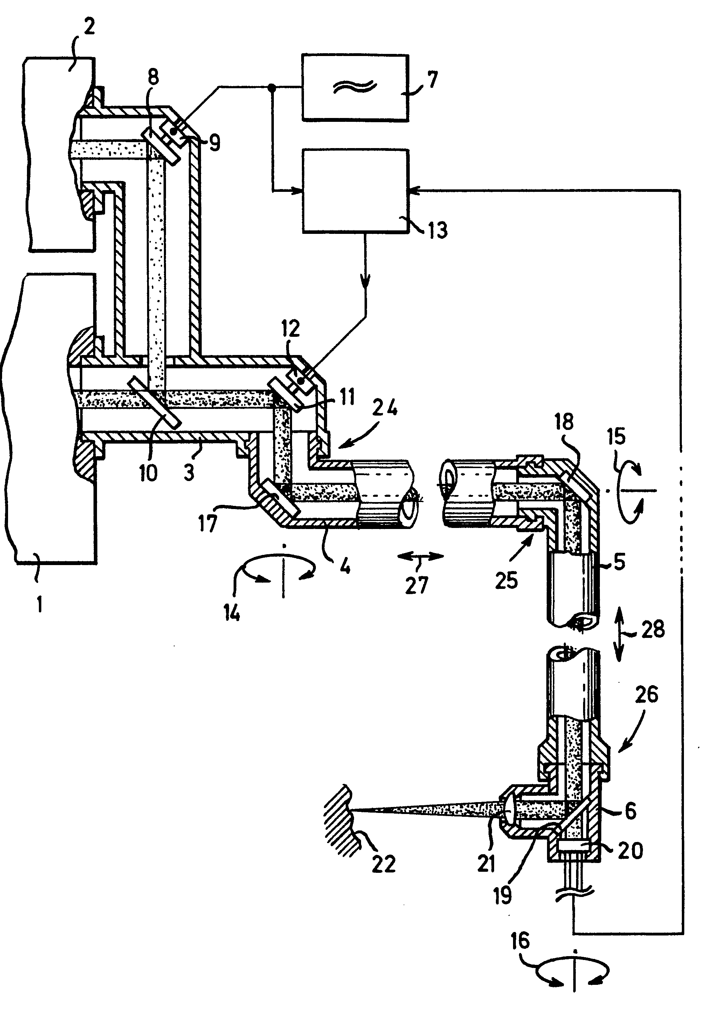

Weitere vorteilhafte Ausgestaltungen der Erfindung ergeben sich aus der nachstehenden Beschreibung der beigefügten Zeichnung, in der ein Ausführungsbeispiel dargestellt ist.

In der Figur der Zeichnung ist mit 1 das Gehäuse eines $CO_2$-Lasers bezeichnet, dessen Strahl über einen Gelenkarm zu einer per Hand in mehreren Freiheitsgraden beweglichen Fakusieroptik 21 geführt ist, mit der der Strahl auf die Oberfläche eines nicht näher spezifizierten Objekts 22 fokussiert wird. Die Gelenkoptik umfasst ein an das Gehäuse 1 angeflanschtes, feststehendes Rohr 3, an dem ein zweites, teleskopisch ausziehbares Rohr 4 drehbeweglich über ein erstes Drehlager 24 befestigt ist, ein drittes ebenfalls ausziehbares Rohr 5, das seinerseits über ein zweites Drehlager 25 am Teil 4 befestigt ist, und den Schneidkopf 6, der die Fakusieroptik 21 enthält und über ein drittes Drehlager 26 am Teil 5 befestigt ist. Durch die Pfeile 14, 15 und 16 wird die Drehrichtung der Lager 24, 25 und 26 angedeutet, während die Pfeile 27 und 28 den Teleskopauszug der Teile 4 und 5 verdeutlichen sollen.

Die Teile 4, 5 und 6 des Gelenksarms sind hinter dem jeweiligen Drehlager 24, 25 und 26, um das sie mit den auf sie folgenden Teilen jeweils drehbar sind, abgewinkelt und enthalten Umlenkspiegel 17, 18 und 19, über die der Laserstrahl im Gelenkarm geführt ist. Ausserdem enthält der gehäusefeste Teil 3 des Gelenkarms einen wellenlängenselektiven Strahlteiler 10, über den koaxial zum Strahl des Arbeitslasers 1 der Strahl eines Pilotlasers 2 in den Gelenkarm eingekoppelt wird. Dieser Pilotlaser 2 dient dazu der Bedienperson, die den Gelenkarm führt, die Position des Fokus des nicht sichtbaren Strahls des Arbeitslasers 1 auf der Oberfläche des Objekts 22 anzuzeigen. Der Spiegel 6, über den der Arbeitslaser 1 in die Fokusieroptik 21 eingespielt wird, ist ebenfalls als Strahlteiler ausgebildet. Er lässt einen Teil der Strahlung des Pilotlasers 2 unreflektiert passieren und damit auf einen dahinter angeordneten, photoelektrischen Quadrantendetektor 20 auffallen.

Die photoempfindliche Fläche des Detektors 20 ist in vier gleich grosse Sektoren aufgeteilt. Die Sektoren dieses Detektors geben Gleichlichtsignale ab, deren Amplituden sich solange nicht voneinander unterscheiden, solange der Strahl des Lasers 2 zentrisch auf den Detektor 20 auftrifft. Sobald der Strahl jedoch auszuwandern beginnt, beispielsweise, weil sich die Teleskopführungen bei Betätigung des Gelenkarmes leicht deformieren oder bedingt durch Schlagfehler in den Drehlagern 24-26, ändern sich die Amplitudenverhältnisse der Gleichlichtsignale der vier Quadranten. Diese Änderung ist ein Mass für den Betrag der Auswanderung des über den Gelenkarm geführten Laserstrahls aus der Achse der Gelenkoptik. Eine elektrische Schaltung 13 wertet die Gleichlichtsignale aus und liefert ein Stellsignal für einen am Eingang des Gelenkarms angeordneten Stellspiegel 11. Mit der Auslenkung des Stellspiegels 11 wird die Auswanderung des Laserstrahls kompensiert.

Der Stellspiegel 11 ist über Piezoelemente 12 am gehäusefesten Teil 13 befestigt. Es ist jedoch klar, dass die Auslenkung des Spiegels 11 auch über z.B. induktive Stellglieder erfolgen kann, wesentlich ist lediglich, dass die Verstellbarkeit des Spiegels 11 in zwei Koordinaten gewährleistet ist.

Da zwischen dem Detektor 20 und dem Stellspiegel 11 jedoch keine starre Verbindung besteht und die Richtung, in der der Spiegel 11 den Laserstrahl auszulenken hat, um die festgestellte Strahlauswanderung zu kompensieren, von dem Drehwinkel der drei Gelenke 24, 25 und 26 abhängt, ist ein zweiter Stellspiegel 8 vorgesehen, der im Strahlengang des Pilotlasers 2 vor dem Strahlteiler 10 angeordnet ist. Dieser Stellspiegel 8 enthält einen piezoelektrischen Biegeschwinger 9, der von einem Generator 7 periodisch erregt wird und den Strahl des Hilfslasers 2 somit bezüglich seiner Strahlrichtung mit kleiner Amplitude moduliert.

Infolge dieser periodischen Modulation der Strahlrichtung des Pilotlasers 2 liefern die Sektoren des Detektors 20 neben dem Gleitlichtsignal ein Wechsellichtsignal, aus dessen Amplitudenverhältnis und Phasenlage in bezug auf das Ausgangssignal des Generators 7 die Drehlage zwischen dem Stellspiegel 11 und dem Detektor 20 bestimmt werden kann. Auch diese Auswertung leistet die elektronische Schaltung 13. Das von ihr abgegebene Stellsignal für den Spiegel 11 verstellt diesen also nach Betrag und Richtung so, dass der durch den Teiler 19 hindurchtretende Teilstrahl des Lasers 2 zentrisch auf den Detektor 20 auftrifft.

Die Schaltung 13 zur Auswertung der Gleich- und Wechsellichtsignale des Detektors 20 lässt sich auf verschiedene Art und Weise realisieren. Die verwendete Ausführungsform hängt beispielsweise davon ab, ob die Modulationsbewegung des Stellspiegels 8 linear oder präzedierend erfolgt.

Ausführungsbeispiele für piezoelektrisch arbeitende Stellspiegel sind u.a. in der DE-PS 2 950 919 beschrieben.

**Patentansprüche**

1. Einrichtung zur Lagekorrektur eines über eine Gelenkoptik geführten Laserstrahls, dadurch gekennzeichnet, dass der Laserstrahl (2) vor Entritt in die Gelenkoptik (17, 18, 19) über ein die Strahlrichtung mit kleiner Amplitude modulierendes, optisches Element (8, 9) geführt, ein Teil der Laserstrahlung auf einen am Ausgang der Gelenkoptik angeordneten, positionsempflindlichen Detektor (20) gerichtet und eine Schaltung (13) vorgesehen ist, der das Signal des Detektors (20) zur Bildung eines Regelsignals für einen am Eingang der Gelenkoptik angeordneten, steuerbaren Strahlablenker (11, 12) zugeführt ist.

2. Einrichtung nach Anspurch 1, dadurch gekennzeichnet, dass das die Strahlrichtung modulierende Element gleichzeitig als Strahlablenker dient.

3. Einrichtung nach Anspruch 1, dadurch gekennzeichnet, dass sich das modulierende optische Element (8, 9) im Strahlengang eines Hilfslasers (2) befindet, der in den Strahl eines Arbeitslasers (1) eingekoppelt ist, und dass beide Laserstrahlen über den steuerbaren Strahlablenker (11, 12) geführt sind.

4. Einrichtung nach Ansprüchen 1-3, dadurch gekennzeichnet, dass als positionsempfindliche Detektoren Quadrantenempfänger verwendet sind.

5. Einrichtung nach Ansprüchen 1-4, dadurch gekennzeichnet, dass als strahlablenkende optische Elemente jeweils piezoelektrisch auslenkbare Stellspiegel verwendet sind.

**Claims**

1. An arrangement for correcting the position of a laser beam guided by means of an articulated optical system, characterized by the fact that the laser beam (2) is guided via an optical element (8, 9) before entering the articulated optical system, which optical element modulates the beam direction with a low modulation amplitude, that part of the laser beam intensity is directed to hit a position sensitive detector (20) being arranged at the output end of the articulated optical system, and that a control unit (13) is provided receiving the signal of said detector (2) and generating a control signal for a beam deflector (11, 12) arranged at the input end of the articulated optical system.

2. The arrangement of claim 1, characterized by the fact that the same optical element is used for modulation of the beam direction and as beam deflector.

3. The arrangement of claim 1, characterized by the fact that the modulating optical element (8, 9) is arranged in the beam path of an auxiliary laser (2), which is coupled into the beam path of a work laser, and that both laser beams are guided via the controllable beam deflector (11, 12).

4. The arrangement according to one of the claims 1-3, characterized by the fact that the position sensitive detectors are quadrant receivers.

5. The arrangement according to one of the claims 1-4, characterized by the fact that the beam deflecting optical elements are piezoelectric adjustable mirrors.

**Revendications**

1. Dispositif de correction de position d'un rayon

laser guidé par l'intermédiaire d'un système optique articulé, caractérisé en ce que le rayon laser (2) est guidé, avant l'entrée dans le système optique articulé (17, 18, 19) par l'intermédiaire d'un élément optique (8, 9) modulant la direction du rayon avec une petite amplitude, une partie du rayonnement laser est dirigée sur un détecteur sensible à une position (20) et disposé à la sortie du système optique articulé et il est prévu un circuit (13) qui reçoit le signal du détecteur (20) de façon à produire un signal de régulation pour un dispositif de déviation de rayon (11, 12) commandé et placé à l'entrée du système optique articulé.

2. Dispositif selon la revendication 1, caractérisé en ce que l'élément modulant la direction de rayon sert simultanément de dispositif de déviation de rayon.

3. Dispositif selon la revendication 1, caractérisé en ce que l'élément optique de modulation (8, 9) est placé dans le trajet du rayon d'un laser auxiliaire (2), qui est interposé dans le rayon d'un laser de travail (1) et en ce que les deux rayons de lasers sont guidés par l'intermédiaire du dispositif de déviation de rayon pouvant être commandé (11, 12).

4. Dispositif selon une des revendications 1 à 3, caractérisé en ce que, comme détecteurs sensibles à une position, on utilise des récepteurs à quadrants.

5. Dispositif selon une des revendications 1 à 4, caractérisé en ce que, comme éléments optiques de déviation de rayon, on utilise respectivement des miroirs de régulation pouvant être déviés piézoélectriquement.